# EUROPEAN PATENT SPECIFICATION

(11) **EP 3 854 181 B1**
(45) Date of publication and mention of the grant of the patent: **07.06.2023**
(21) Application number: 19828847.4
(22) Date of filing: 20.09.2019
(51) Int. Cl.: H05H 1/24, A61N 1/44, A61B 18/04, A61L 2/14

(54) **ELECTRODE ARRANGEMENT FOR A DBD PLASMA TREATMENT**
ELEKTRODEANORDNUNG FÜR EINE DBE-PLASMABEHANDLUNG
AGENCEMENT D'UNE ELECTRODE POUR UN TRAITEMENT PLASMA PAR DECHARGE À BARRIERE DIÉLECTRIQUE

(30) Priority: 20.09.2018 NL 2021675
(43) Date of publication of application: 28.07.2021
(73) Proprietor: Plasmacure B.V., 6534 AT Nijmegen (NL)
(72) Inventor: ZEPER, Wouter Bastiaan, 6534 AT Nijmegen (NL); SMITS, Paulien, 6534 AT Nijmegen (NL); DE PENNING, Johannes Pieter, 6534 AT Nijmegen (NL); VAN OORT, Matthijs Andreas, 6534 AT Nijmegen (NL)
(74) Representative: V.O.
(86) International application number: PCT/NL2019/050629
(87) International publication number: WO 2020/060409

(56) References cited:
- WO-A2-2016/186501
- DE-A1- 19 816 377
- US-A1- 2001 013 759
- US-A1- 2015 209 595
- US-A1- 2016 166 818

## Description

### FIELD OF THE INVENTION

This invention relates to devices for generating non-thermal plasma. In particular, the invention relates to devices that can be applied for treatment of living tissue.

### BACKGROUND OF THE INVENTION

Cold plasmas have considerable potential for skin conditioning, disinfection of skin and wound healing. This is due to the reactive species as well as the electric field that are produced. The skin will be temporary exposed to the plasma to kill microorganisms and to stimulate the human skin and immune cells (e.g. improve cell proliferation) and the microcirculation of the blood.
A problem related to the plasma discharge, is that, due to energy absorption in the skin, it is not possible to full-time continue with plasma discharge. Thus, a pulsed discharge is provided. It is an aim to more efficiently use the electric field, without compromising the treated tissue due to over exposure. An example of a device for the generation of cold plasma for skin treatment by means of electrical pulses is disclosed in WO2016/186501 A2.
US2016/0166818 A1 and US2015/0209595 A1 also relate to the use of electrical pulses tot generate a cold plasma for the treatment of biological surfaces. Cold plasma may also be used in discharge lamps, as disclosed in US2001/0013759 A1, or in application related to printing, as disclosed in DE 198 16 377 A1.

### SUMMARY OF THE INVENTION

In accordance with claim 1, the invention pertains to an electrode arrangement for a dielectric barrier discharge plasma treatment of a to be treated tissue of a patient, which treatment surface is used as a counter electrode, having
- a plasma generating electrode to be coupled to high voltage source via a first lead;
- a dielectric shielding the plasma generating electrode from the surface to be treated;
- a spacer defining a structured surface on a side of said electrode arrangement facing a surface to be treated,
- said plasma generating electrode being fitted to the object to be treated and brought in contact with the dielectric,
- a high voltage driver circuit for driving the plasma generating electrode, wherein the high voltage driver circuit drives the plasma generating electrode in first periods wherein a first voltage is applied to the plasma generating electrode; said driver being further arranged to drive the plasma generating electrode in second periods wherein a second voltage is applied to the plasma generating electrode, wherein both first and second voltages do not exceed a range of 3-8kV and wherein the first voltage creates a dielectric barrier discharge plasma to the treatment surface; and the second voltage does not create a dielectric barrier discharge plasma. The first voltage may produce reactive species and electric fields that kill microbes and/or stimulate human cells, to disinfect skin and/or aid wound healing. The second voltage may produce only electric fields that kill microbes and/or stimulate human cells. These effects can be promoted by plasmas and electric fields with different intensity and HV pulse durations. In an embodiment, in an off-period, the plasma and electric fields fully disappear, and the thermal energy can be dissipated. The plasma treatment may thus be given in multiple intervals with an intermediate period without plasma or electric fields to give time to dissipate the heat produced by the plasma in order to prevent heating up of the skin to an uncomfortable or damaging level. The heat is produced only during the time the plasma or electric fields are present, induced by the power. Since there is no plasma or electric fields present during the off-period, the treatment is not fully effective during the treatment time. Some of the reactive species formed by the plasma will still be present during the off-period, but the electric field will be fully absent.
Preferred embodiments of the invention are defined in the dependent claims.

### BRIEF DESCRIPTION OF THE DRAWINGS

Figure 1 shows a schematic perspective view of a system configuration of the cold plasma device;
Figure 2 shows a first schematic example of a new voltage control scheme for a high voltage driving circuit;
Figure 3 shows a first schematic example of a high voltage driving circuit;
Figure 4 shows an exemplary schematic for the PWM (pulse width modulation) pulse inputs of the high voltage driving circuit; and
Figure 5 shows an exemplary form of the generated voltages on the load for the PWM pulse inputs of Figure 4.

### DETAILED DESCRIPTION

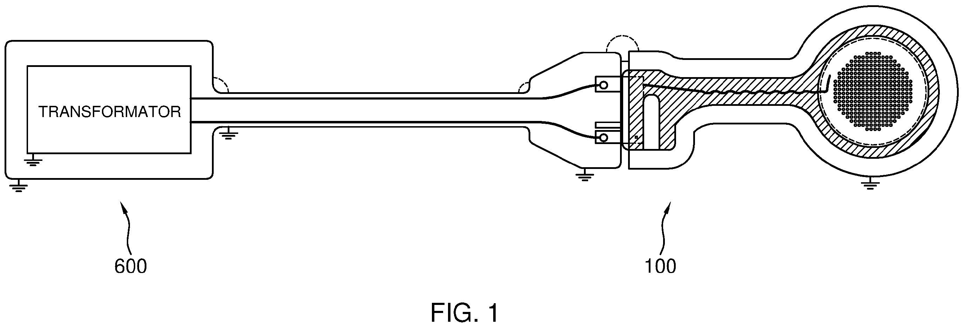

Figure 1 shows a schematic perspective view of a prototype of the cold plasma device. The plasma device 100 provides a dielectric barrier discharge (DBD) technique for plasma generation. The plasma can be powered by repetitive, short high-voltage (HV) pulses (0.1 ns- 10 ms duration, up to a few 100 kHz repetition rate). For example, a driver circuit 600 is provided for driving the planar electrode, wherein the driver circuit drives the planar electrode in a pulsed voltage in a range of 3-8kV, in a range of 1-100Hz, and a PWM pulse duration in a range of 50 -150 micro seconds. This allows for a pulse rate that substantially provides a micro discharge wherein electrical current through the object to be treated (skin, human body) will only flow during the time that the plasma is on (which is typically equal to the HV pulse duration). In between the HV pulses, the plasma is not active, and no substantial current flows through the skin.

A DBD cold plasma device can treat large areas; the dimensions of the DBD can be chosen over wide margins. Instead of allowing for airflow between the cold plasma device and the skin, discrete compartments may be formed that will contain some air, but these need not be connected to each other. They may be isolated from each other, and may also be isolated to the surroundings by a closed edge.

The advantage of a closed compartment is that the reactive gases that we will generate during operation of the cold plasma, gases like ozone, cannot escape. This has the advantage that the device is more efficient: all reactive specimens are available to kill pathogens and stimulate human cells, and that the release of any toxic gases like ozone will be minimized.

Accordingly an electrode arrangement 100 is shown for a dielectric barrier discharge plasma treatment of an irregularly three-dimensionally shaped surface of an electrically conducting body. The body is typically a human body part, such as a heel, toe, finger or any other diseased skin part, which surface is used as a counter electrode.
The arrangement has a first planar electrode 1 to be coupled to a high voltage source; a dielectric is formed by a flexible material in such a way that the dielectric shields the first planar electrode from the surface to be treated. A spacer structure defines a structured surface on a side of said arrangement 100 facing a surface to be treated.

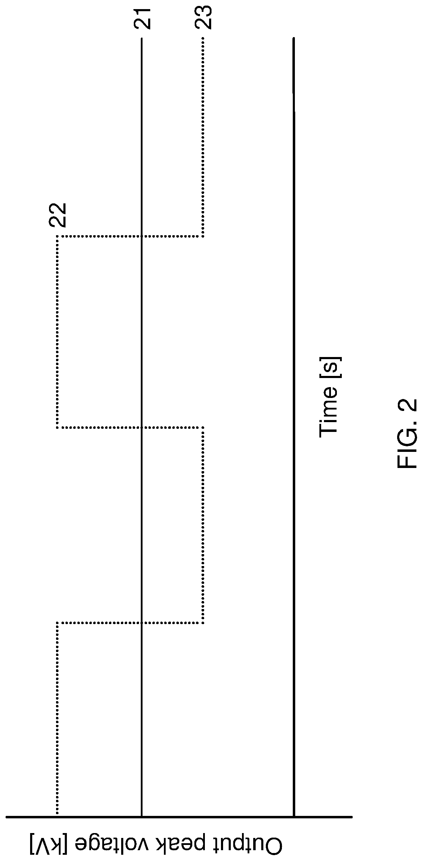

Figure 2 shows an illustrative output voltage scheme, where it is found that first PWM pulses have a duration of e.g. 50-150 microseconds, with a repetition cycle of e.g. 10 millisecond, e.g. operating on a frequency of 100 Hz, to generate a high voltage (22) above the level needed for DBD discharge (21). Similarly, in the second period, second PWM pulses are generated having a duration of e.g. 5 microsecond- 100 microsecond at a repetition rate of 500-1500 Hz. The circuit has characteristics so that this results in a second voltage (23) at a lower level than the first level, typically too little to generate a discharge, but sufficient to generate electric fields up to 10^5 V/m.

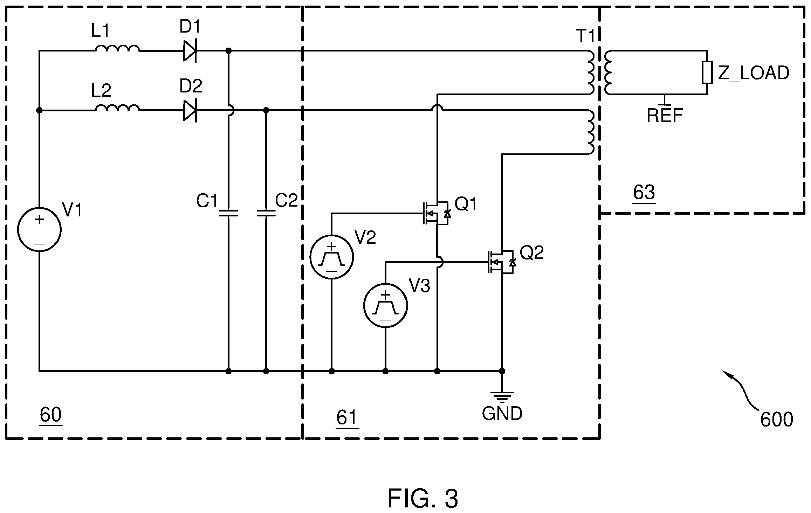

Figure 3 shows an example driving circuit 600 for driving the plasma generating (ZLoad) in first periods wherein a first voltage is applied to the plasma generating and driving the plasma generating in second periods wherein a second voltage is applied to the plasma generating. The example driving circuit 600 is structured as a pulsed high voltage converter. While various examples can be contemplated and are deemed to be covered by the present disclosure, the circuit generally operates on a power circuit 60 that releases electrical energy into a second control circuit 61, that is accumulated during an active pulse period wherein the electrical energy from a source V1 is inserted in a transformer T1 by a PWM controller V2 and transferred to a third circuit 63 including a secondary winding of the transformer T1 and the load of the plasma generating. To make it possible to generate HV pulses with different pulse durations an extra control element V3 is added in the driving circuit. The hardware can switch between the two PWM driving components V2 and V3 to get an output with multiple pulse durations. The different HV pulse durations are used, in the first and second periods, to obtain different biological effects. In the first 'plasma' period, electric fields above 10^5 V/m and HV pulse durations e.g. of 10^-10 - 10^-2 seconds can result in either intracellular manipulation, irreversible electroporation or reversible electroporation, depending on the exact electric field and HV pulse duration as well as the size of the biological cell. In the second `electric field' period, electric fields up to 10^5 V/m and HV pulse durations e.g. of 10^-6 - 10^-2 seconds can result in irreversible or reversible electroporation, again depending on the factors mentioned before. It is noted that these biological effects are in itself known e.g. from Intense picosecond pulsed electric fields induce apoptosis through a mitochondrialmediated pathway in HeLa cells, Yuan-Yuan Hua et al., DOI 10.3892/mmr.2012.780.

The power circuit 60 includes a power capacitor coupled with the primary winding of the transformer T1. In control circuit 61, a first controllable conductor Q 1 is coupled in series to provide a pulsed primary current in the primary winding resonating with the capacitor C1 when the first controllable conductor is switched in a conducting on-state. When the first controllable conductor Q1 is switched in a non conducting off-state the capacitor C1 is fed with electrical current from the voltage source V1.

In the illustrated form, the first power circuit 60 is formed by two power capacitors C1 and C2 in dual circuits each having a diode for unidirectional current flow. The two circuits each generate a different electrical power for driving the second circuit 61 including transformer T1, where the power of the L1C1 circuit is coupled via a first primary winding, and the power of the L2C2 circuit is coupled via a second primary winding of the transformer T1, resulting in two different waveforms in the third circuit 63, needed for two different HV pulse durations.

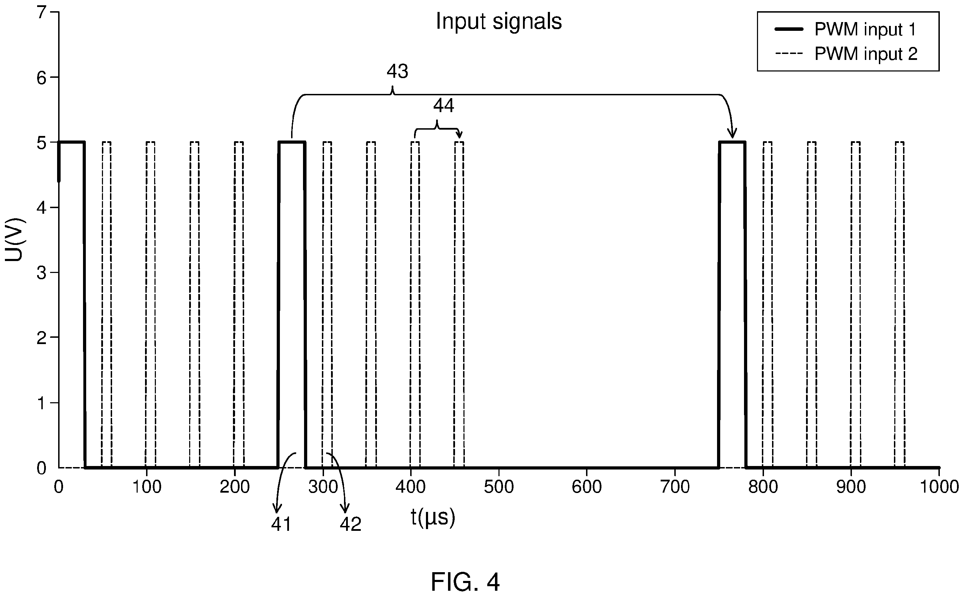

Figure 4 illustrates an exemplary PWM pulse scheme for driving the high voltage driver circuit of the type of Figure 3. It is possible to provide a quieter configuration by applying an intermediate field that is provided by second PWM pulses (42) of shorter duration on one of controllable conductors Q1, Q2 of the control circuit 61. The shorter PWM pulses have a repetition rate (44) of about 0.5-1.5 kHz and have a higher repetition rate than first PWM pulses (41) with a longer duration and a lower repetition rate (43) of about 1-100 Hz that is provided by the controller on the other of the controllable conductors Q1, Q2 to control the discharge of the power circuit. As the used voltage for breakdown can be lowered to a lower repetition rate, an accompanying buzzing sound is rendered less audible. To prevent loud and annoying noise during treatment with 1 kHz plasma, the pulse frequency is thus divided into two frequencies: a high frequency, for example 1 kHz and low frequency, for example 100 Hz. The high frequency pulses have a lower voltage so that only an electric field and barely any sound is generated. The low frequency pulses are pulses with plasma and sound, but the sound will be less loud and not annoying due to the lower frequency. Additionally, the electromagnetic radiation produced by the plasma will be reduced.

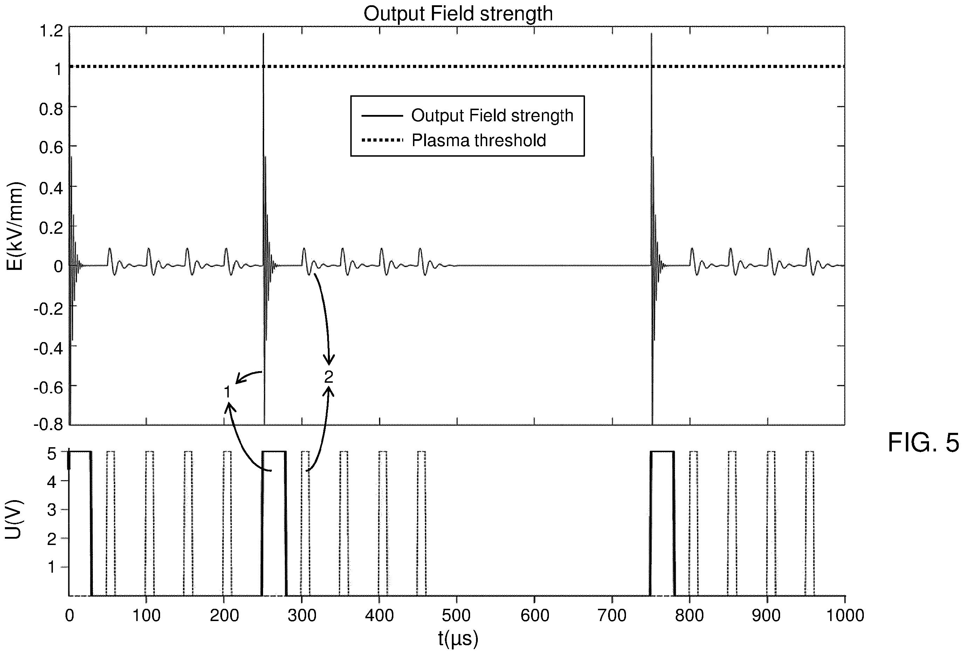

Figure 5 shows the input plotted simultaneous with a corresponding output in the third circuit, i.e. the voltage measured over the load - being the plasma generating electrode. It is shown that the first PWM pulses (1) of longer durations correspond with the plasma generating voltage peaks of higher voltages (e.g. higher than 10^5 V/m) - where the second PWM pulses (2) of shorter duration correspond to voltage peaks up to 10^5 V/m. The relation between voltage output and PWM pulse duration may correspond to circuit specific details and may vary, as long as the PWM pulse duration is long enough to create a high enough voltage to create a plasma.

### Further embodiments

The method to use the cooling down time effectively is to produce a non-igniting electric field on the electrode by applying a lower voltage than the normal operation voltage on the pad (3). The intermediate period is now used for cooling down as well as for continued stimulation of human cells by applying a continuous electric field.

Intervals and duration of the plasma can be determined by: a fixed program; or based on a measurement, e.g. temperature measurement or reactive species measurement. In this way, a dynamic signal modulation is used to control different operation modes during a treatment. This is used to control the temperature during plasma treatment while maximizing the treatment efficiency and thereby the effectiveness. And using varying frequency to reduce noise. The dual circuits make HV pulse duration variations possible for a single device.

## Claims

1. An electrode arrangement (100) for a dielectric barrier discharge plasma treatment of a to be treated tissue of a patient, which treatment surface is used as a counter electrode, having
- a plasma generating electrode (1) to be coupled to a high voltage source via a first lead;
- a dielectric shielding the plasma generating electrode (1) from the surface to be treated;
- a spacer defining a structured surface on a side of said electrode arrangement facing a surface to be treated,
- said plasma generating electrode being fitted to the object to be treated and brought in contact with the dielectric,
- a high voltage driver circuit (600) for driving and coupled to the plasma generating electrode , wherein the high voltage driver circuit (600) is arranged to drive the plasma generating electrode (1) in first periods wherein a first voltage (22, 41) is applied to the plasma generating electrode (1); and wherein said driver is further arranged to drive the plasma generating electrode (1) in second periods wherein a second voltage (23, 42) is applied to the plasma generating electrode (1), wherein both first and second voltages (22, 41; 23,42) do not exceed a range of 3-8kV and **characterized in that** the first voltage (22,41) in the first periods creates a dielectric barrier discharge plasma; and the second voltage (23, 42) in the second periods does not create a dielectric barrier discharge plasma.

2. The electrode arrangement (100) according to claim 1, wherein the high voltage driver circuit (600) is arranged to provide in the first and second periods, respectively, a first HV pulse duration differing from a second HV pulse duration.

3. The electrode arrangement (100) according to claim 2, wherein the high voltage driver circuit (600) is arranged to provide in the first and second periods a HV pulse duration in a range of 0.1 nano second - 10 milli seconds.

4. The electrode arrangement (100) according to any preceding claim, wherein the high voltage driver circuit (600) is equipped with pulse width modulated sources arranged to provide the second voltage (42) with a repetition rate and/or PWM pulse duration differing from the repetition rate (44) and/or PWM pulse duration of the first voltage (41).

5. The electrode arrangement (100) according to claim 4, wherein the high voltage driver circuit (600) is arranged to provide the first voltage (41) with the first repetition rate (43) in a range of 1-100 Hz, and a PWM pulse duration in a range of 50 -150 micro seconds.

6. The electrode arrangement (100) according to claim 5, wherein the high voltage driver circuit is arranged to provide the second voltage in the second periods at a second pulsed frequency that do not overlap the first periods of the first voltage.

7. The electrode arrangement (100) according to claim 6, wherein the second voltage (42) is pulsed at a frequency in the 0.5-1.5 kHz range, and a PWM pulse duration in a range of 5 - 100 micro seconds.

8. The electrode arrangement (100) according to any preceding claim, wherein the high voltage driver circuit (600) is arranged to provide an off-period where no voltage is supplied, the off-period being alternating with the first or second period.

## Patentansprüche

1. Elektrodenanordnung (100) für eine dielektrische Barriereentladungsplasmabehandlung eines zu behandelnden Gewebes eines Patienten, wobei die Behandlungsfläche als Gegenelektrode verwendet wird, die aufweist
- eine plasmaerzeugende Elektrode (1), die über eine erste Leitung mit einer Hochspannungsquelle zu koppeln ist;
- ein Dielektrikum, das die plasmaerzeugende Elektrode (1) von der zu behandelnden Oberfläche abschirmt;
- einen Abstandshalter, der eine strukturierte Oberfläche auf einer Seite der Elektrodenanordnung definiert, die einer zu behandelnden Oberfläche gegenüberliegt,
- wobei die plasmaerzeugende Elektrode an dem zu behandelnden Objekt angebracht und mit dem Dielektrikum in Kontakt gebracht wird,
- eine Hochspannungstreiberschaltung (600) zum Treiben der und Koppeln mit der plasmaerzeugenden Elektrode, wobei die Hochspannungstreiberschaltung (600) so angeordnet ist, dass sie die plasmaerzeugende Elektrode (1) in ersten Perioden treibt, in denen eine erste Spannung (22, 41) an die plasmaerzeugende Elektrode (1) angelegt wird; und wobei der Treiber ferner so angeordnet ist, dass er die plasmaerzeugende Elektrode (1) in zweiten Perioden treibt, wobei eine zweite Spannung (23, 42) an die plasmaerzeugende Elektrode (1) angelegt wird, wobei sowohl die erste als auch die zweite Spannung (22, 41; 23, 42) einen Bereich von 3 bis 8 kV nicht überschreiten, und **dadurch gekennzeichnet, dass** die erste Spannung (22, 41) in den ersten Perioden ein dielektrisches Barrierenentladungsplasma erzeugt; und die zweite Spannung (23, 42) in den zweiten Perioden kein dielektrisches Barrierenentladungsplasma erzeugt.

2. Elektrodenanordnung (100) nach Anspruch 1, wobei die Hochspannungstreiberschaltung (600) so angeordnet ist, dass sie in der ersten und zweiten Periode eine erste HV-Pulsdauer bereitstellt, die sich von einer zweiten HV-Pulsdauer unterscheidet.

3. Elektrodenanordnung (100) nach Anspruch 2, wobei die Hochspannungstreiberschaltung (600) so angeordnet ist, dass sie in den ersten und zweiten Perioden eine HV-Pulsdauer im Bereich von 0,1 Nanosekunden bis 10 Millisekunden bereitstellt.

4. Elektrodenanordnung (100) nach einem der vorhergehenden Ansprüche, wobei die Hochspannungstreiberschaltung (600) mit pulsbreitenmodulierten Quellen ausgestattet ist, die so angeordnet sind, dass sie die zweite Spannung (42) mit einer Wiederholungsrate und/oder PWM-Pulsdauer bereitstellen, die sich von der Wiederholungsrate (44) und/oder PWM-Pulsdauer der ersten Spannung (41) unterscheidet.

5. Elektrodenanordnung (100) nach Anspruch 4, wobei die Hochspannungstreiberschaltung (600) so angeordnet ist, dass sie die erste Spannung (41) mit der ersten Wiederholungsrate (43) in einem Bereich von 1 bis 100 Hz und einer PWM-Pulsdauer in einem Bereich von 50 bis 150 Mikrosekunden bereitstellt.

6. Elektrodenanordnung (100) nach Anspruch 5, wobei die Hochspannungstreiberschaltung so angeordnet ist, dass sie die zweite Spannung in den zweiten Perioden mit einer zweiten gepulsten Frequenz bereitstellt, die sich nicht mit den ersten Perioden der ersten Spannung überschneiden.

7. Elektrodenanordnung (100) nach Anspruch 6, wobei die zweite Spannung (42) mit einer Frequenz im Bereich von 0,5 bis 1,5 kHz und einer PWM-Pulsdauer im Bereich von 5 bis 100 Mikrosekunden gepulst wird.

8. Elektrodenanordnung (100) nach einem der vorhergehenden Ansprüche, wobei die Hochspannungstreiberschaltung (600) so angeordnet ist, dass sie eine Aus-Periode bereitstellt, in der keine Spannung zugeführt wird, wobei sich die Aus-Periode mit der ersten oder zweiten Periode abwechselt.

## Revendications

1. Agencement d'électrodes (100) pour un traitement par plasma à décharge à barrière diélectrique d'un tissu à traiter d'un patient, laquelle surface de traitement est utilisée comme contre-électrode, ayant
- une électrode génératrice de plasma (1) couplée à une source de haute tension via un premier fil ;
- un diélectrique protégeant l'électrode génératrice de plasma (1) de la surface à traiter ;
- une entretoise définissant une surface structurée sur un côté dudit agencement d'électrodes faisant face à une surface à traiter,
- ladite électrode génératrice de plasma étant montée sur l'objet à traiter et mise en contact avec le diélectrique,
- un circuit d'attaque haute tension (600) pour l'attaque, couplé à l'électrode génératrice de plasma, dans lequel le circuit d'attaque haute tension (600) est agencé pour attaquer l'électrode génératrice de plasma (1) au cours de premières périodes où une première tension (22, 41) est appliquée à l'électrode génératrice de plasma (1) ; et dans lequel ledit circuit d'attaque est en outre agencé pour attaquer l'électrode génératrice de plasma (1) au cours de deuxièmes périodes où une deuxième tension (23, 42) est appliquée à l'électrode génératrice de plasma (1), dans lequel les première et deuxième tensions (22, 41 ; 23, 42) ne dépassant pas toutes deux une plage de 3 à 8 kV, et **caractérisé en ce que** la première tension (22, 41) au cours des premières périodes crée un plasma de décharge à barrière diélectrique ; et **en ce que** la deuxième tension (23, 42) au cours des deuxièmes périodes ne crée pas de plasma de décharge à barrière diélectrique.

2. Agencement d'électrodes (100) selon la revendication 1, dans lequel le circuit d'attaque haute tension (600) est agencé pour fournir, respectivement au cours des première et deuxièmes périodes, une première durée d'impulsion HV différente d'une deuxième durée d'impulsion HV.

3. Agencement d'électrodes (100) selon la revendication 2, dans lequel le circuit d'attaque haute tension (600) est agencé pour fournir, au cours des première et deuxièmes périodes, une durée d'impulsion HV dans une plage de 0,1 nano seconde à 10 milli secondes.

4. Agencement d'électrodes (100) selon l'une quelconque des revendications précédentes, dans lequel le circuit d'attaque haute tension (600) est équipé de sources modulées en largeur d'impulsion agencées pour fournir à la deuxième tension (42) un taux de répétition et/ou une durée de l'impulsion PWM qui diffère du taux de répétition (44) et/ou de la durée de l'impulsion PWM de la première tension (41).

5. Agencement d'électrodes (100) selon la revendication 4, dans lequel le circuit d'attaque haute tension (600) est agencé pour fournir à la première tension (41) le premier taux de répétition (43) dans une plage de 1 à 100 Hz, et une durée de l'impulsion PWM dans une plage de 50 à 150 micro secondes.

6. Agencement d'électrodes (100) selon la revendication 5, dans lequel le circuit d'attaque haute tension est agencé pour fournir la deuxième tension au cours des deuxièmes périodes à une deuxième fréquence en impulsion qui ne chevauche pas les premières périodes de la première tension.

7. Agencement d'électrodes (100) selon la revendication 6, dans lequel la deuxième tension (42) est pulsée à une fréquence dans la plage de 0,5 à 1,5 kHz, et une durée de l'impulsion PWM est dans une plage de 5 à 100 micro secondes.

8. Agencement d'électrodes (100) selon l'une quelconque des revendications précédentes, dans lequel le circuit d'attaque haute tension (600) est agencé pour fournir une période de désactivation où aucune tension n'est fournie, la période de désactivation s'alternant avec la première ou la deuxième période.
